# EUROPEAN PATENT APPLICATION

(11) **EP 3 165 919 A1**
(43) Date of publication of application: **10.05.2017**
(21) Application number: 16196850.8
(22) Date of filing: 02.11.2016
(51) Int. Cl.: G01N 33/18, G01N 33/28, G01N 21/05, G01N 21/15, G01N 21/85, G01N 1/20

(54) **EASILY INSTALLABLE AND REMOVABLE FLOW ANALYZER FOR HARSH ENVIRONMENTS**

(30) Priority: 02.11.2015 US 201562249391 P; 01.11.2016 US 201615339991
(71) Applicant: J.M. Canty Inc., Buffalo, NY 14094 (US)
(72) Inventor: Canty, Thomas M., 14221 Amherst, New York (US); O'Brien, Paul J., 14052 East Aurora, New York (US); Marinelli, Richard F., 14223 Tonawanda, New York (US)
(74) Representative: DREISS Patentanwälte PartG mbB

(57) **Abstract**

A flow analysis system for analyzing fluid from a process flow line includes a flow analyzer, a single point sampler configured to connect to the process flow line, the single point sampler including a sample line having an inflow passage and an outflow passage, an inflow pipe connecting the inflow passage of the sample line to the flow analyzer, and an outflow pipe connecting the flow analyzer to the outflow passage of the sample line.

## Description

### CROSS REFERENCE TO RELATED APPLICATIONS

This application claims benefit to U.S. Provisional Patent Application No. 62/249,391, filed November 2, 2015 and to U.S. Non-Provisional Patent Application No. 15/339,991, filed November 1, 2016.

### FIELD

The application relates to devices for analyzing the contents of a process pipeline or vessel, and more particularly to devices for analyzing the contents of a process pipeline or vessel that are capable of being easily installed and removed in harsh environments, e.g. subsea environments.

### BACKGROUND

In certain industries, it is desirable to observe and analyze the contents within an enclosed pipeline or vessel without substantially disrupting the flow or process therein. For example, U.S. Pat. No. 6,771,366, which is incorporated by reference herein in its entirety, describes a flow cell device system that includes an optical flow cell that enables automatic visual analysis and inspection of fluids for various characteristics including particle size, shape, color, and count, among others. The system includes two viewing ports, each containing a transparent glass window to allow illumination and/or viewing of a fluid in an aperture defined between the two viewing ports.

Advances in such flow cells have been made over time. For example, U.S. Pat. Nos. 6,782,184 and 8,297,302, which are incorporated by reference herein in their entirety, describe spray ring devices that provide for cleaning of an internal process window. These spray rings are configured to discharge a fluid onto the window surface in order to dislodge any particles or substances on the window that are hindering observation of the flow through the flow cell.

In connection with extracting crude oil from offshore locations, it is desirable to perform the process of separating crude oil from water, hydrates, and solids at subsea, e.g., sea floor locations. Performing crude oil separation processes at subsea locations could both lower the costs of and reduce environmental safety risks associated with offshore oil extraction. In particular, performing crude oil separation at subsea locations could eliminate the need to locate certain production and processing equipment on floating vessels exposed to violent weather events. Furthermore, performing crude oil separation at subsea locations could reduce production costs associated with offshore extraction. By removing undesired waste components (e.g. water, hydrates, solids) from the extracted mixture at subsea locations and transporting mostly pure crude oil to the surface, costs associated with transporting such undesired waste components to the surface are eliminated.

### SUMMARY

In an embodiment, the present invention provides a flow analysis system for analyzing fluid from a process flow line. The system includes a flow analyzer, a single point sampler configured to connect to the process flow line, the single point sampler including a sample line having an inflow passage and an outflow passage, an inflow pipe connecting the inflow passage of the sample line to the flow analyzer, and an outflow pipe connecting the flow analyzer to the outflow passage of the sample line.

### BRIEF DESCRIPTION OF THE DRAWINGS

The present invention will be described in even greater detail below based on the exemplary figures. The invention is not limited to the exemplary embodiments. All features described and/or illustrated herein can be used alone or combined in different combinations in embodiments of the invention. The features and advantages of various embodiments of the present invention will become apparent by reading the following detailed description with reference to the attached drawings which illustrate the following:
FIG. 1 shows a schematic illustration of an example use of a flow analyzer according to an embodiment of the invention in a harsh environment;
Fig. 2 shows a flow analysis system according to an embodiment of the invention including a flow analyzer connected with a sampler via multiple shut off valves;
Fig. 3 shows a flow analyzer connected to a main flow line via hot stab connectors, shut off valves, and a sampler in accordance with an embodiment of the invention; and.
Fig. 4 shows a hot-stab connector according to an embodiment of the invention.

### DETAILED DESCRIPTION

Performing crude oil separation processes at subsea locations requires that the analysis of process pipeline contents be performed at subsea locations. Flow content analyzers capable of withstanding the harsh environmental conditions present at subsea locations are necessary for performing process pipeline content analysis at subsea locations, and therefore, such flow content analyzers are necessary for enabling separation processes to be performed at subsea locations. In order to withstand the harsh environmental conditions present at subsea locations, the connections between components of analyzer devices as well as the connections between the analyzer and the process pipeline must be robust and capable of withstanding the extreme pressures and other harsh conditions present at the seafloor.

Furthermore, installation and removal of pipeline content analyzers to and from pipelines located at subsea, e.g., seafloor, locations can prove difficult. Beyond certain depths, divers are not able to withstand or survive subsea conditions. Therefore, it is necessary to use remote operated vehicles to install and retrieve pipeline analysis instruments. For example, manned or unmanned submarines or submersible remote operated vehicles (ROV) may be required for the installation and removal of process pipeline content analyzers beyond certain depths. However, as such submersible machines exhibit limited dexterity, it is beneficial to design and construct pipeline content instrumentation for ease installation and removal. Ease of installation and removal from a process pipelines reduces the risk of complications arising during the manipulation of such instrumentation by submersible machinery thereby enhancing the reliability of the entire subsea separation process.

In an embodiment, the present invention provides a flow analysis system that includes a sampler, e.g. a single point sampler, for delivering process fluid from a process flow line. The single point sampler can be, e.g., a single point sampling loop, an inline wafer sampler, a metallic wafer style sampling valve, and an annular probe. The flow analysis system delivers the process fluid taken from the single point sampler to a content analysis instrument. In particular, the single point sampler draws fluid off from the process flow line and sends it to the content analysis instrument. In the case of an annular probe single point sampler, the flow is plumbed back into the center of the probe and emptied back into the process flow line after the flow has gone around the loop.

The content flow analysis instrument can be, for example, an oil in water analyzer. The content flow analysis instrument can be constructed with a reinforced structure sufficient to withstand the conditions of a subsea environment at depths of up to 15,000 feet. The content flow analysis instrument may include one or more spray rings for cleaning internal lenses.

The content flow analysis instrument can be attached to the sampler via one or more hot stab connectors. The hot stab connectors are a male and female two-piece type pipe connector. When the male and female pieces are connected, the hot stab connectors prove a flow path, e.g. for process fluid, that is sealed off from the outside environment. The flow path provided by the connector can be arranged at an angle of ninety degrees to the direction in which the male piece engages with the female piece. This eliminates the need for a force to be applied in the axial direction in order to retain the fitting of the male and female pieces. The hot-stab connectors can be designed and constructed for attachment and removal by a submersible remote operated vehicle (ROV).

In addition, the content flow analysis instrument can be attached to the sampler via one or more shut off valves. The shut off valves can be configured to open when the content flow analysis instrument has been engaged via the one or more hot stab connectors to complete an analysis flow loop line. The shut off valves can also be configured to be closed when the content flow analyzer is disengaged from its connectors.

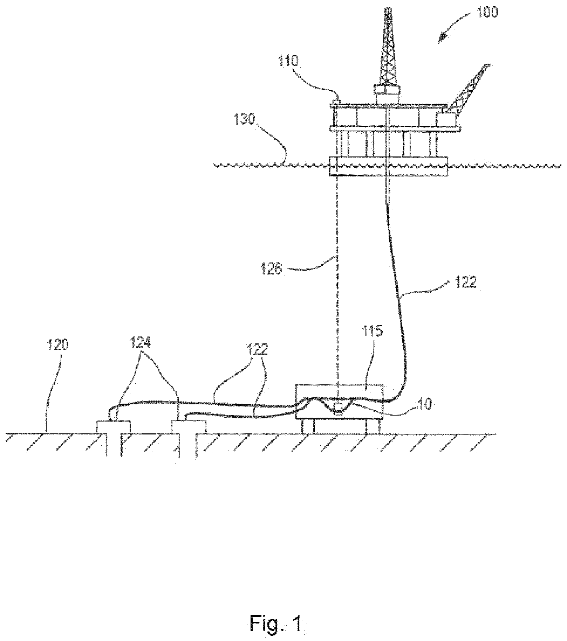

Fig. 1 shows a schematic illustration of an example use of a flow analyzer in a harsh environment. Fig. 1 depicts a flow analyzer 10 disposed within harsh environmental conditions that analyzes the contents of a fluid flow and sends the corresponding analytical data to a communications device 110 at a base station 100 located above the water surface 130. In the embodiment of Fig. 1, the flow analyzer 10 is disposed at a remote distance from the base station 100. In the context of this disclosure, the term "remote" is used to indicate a distance of at least one hundred feet, and including distances of several hundred feet to one or more miles. Of course, the flow analyzer 10 could also be disposed nearer to the base station 100, which itself may also be located at the sea floor.

In the illustrated embodiment shown in Fig. 1, the flow analyzer 10 is configured as a subsea analyzer and is located in a harsh underwater environment. To accommodate these conditions, the analyzer 10 is adapted to operate at the extreme pressure and corrosive conditions existing near the sea floor 120. The flow analyzer 10 in Fig. 1 is specifically configured to analyze the fluid flow in flow lines 122 extending from at least one subsea well 124. Although Fig. 1 shows two subsea wells 124, any number of subsea wells is contemplated herein, with flow lines running to each well. The analyzer 10 is positioned near the opening of the well 124 and analyzes a portion of the flow passing through the flow line 122. In some embodiments, such as the embodiment illustrated in Fig. 1, the analyzer 10 can be disposed within a subsea manifold structure 115, which can rest on the sea floor 120. The subsea manifold 115 can act as a separation module for the flow lines 122 connected to the well heads 124, and can also house the analyzer 10. It is also contemplated, however, that in some embodiments, the analyzer 10 can be connected directly to the flow lines 122 without being housed in a subsea manifold.

In this embodiment, the base station 100 is a facility intended to extract and process oil and/or natural gas from the wells 124. For example, the base station 100 is specifically depicted as a semi-submersible drilling rig, but could also be a similar facility located on a ship. The station could also be located on the sea floor. In the subsea environment depicted, the base station 100 is disposed in fluid communication with the flow line 122. Specifically, the fluid in the flow line 122 is flowing to the base station 100. Likewise, the analytical data from the flow analyzer 10 is sent to the base station 100, where it is received by the communications device 110. Similarly, in many other embodiments, the base station 100 will be disposed in communication with the flow line 122 of interest, either at the source of the flow or at the destination of the flow. Alternatively, the base station 100 may be physically separated from the flow line 122 and merely receive data from the flow analyzer 10. For example, in the context of the subsea analyzer 10 shown in Fig. 1, the base station 100 could be disposed on the shore or on a central control ship that oversees the operations of various extraction and processing facilities. In a preferred embodiment of the subsea configuration, the base station 100 is at or above the water surface 130, such as the semi-submersible oil platform shown in Fig. 1.

The flow analyzer 10 includes a control system 50 that will be described in more detail below. In the embodiment shown in Fig. 1, the control system of the flow analyzer includes a data output for establishing a communications link 126 with a data input of the communications device 110 on the base station 100. Using the communications link 126, the flow analyzer 10 is able to send analytical data representative of the flow to the communications device 110 on the base station 100. The communications link 126 can be established with a physical connection between the flow analyzer 10 and communications device 110, for example using a wired connection or fiber optic connection, or it may be established with a wireless connection.

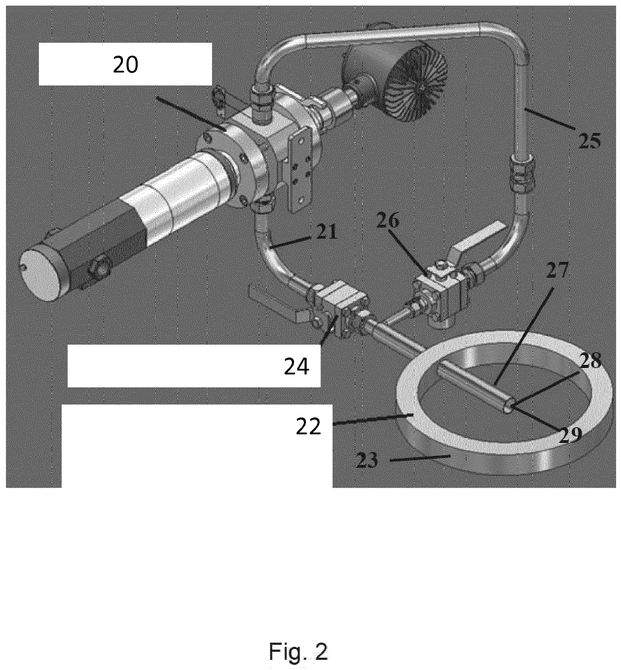

Fig. 2 shows a flow analysis system including a flow analyzer connected with a sampler via multiple shut off valves. In the embodiment depicted in FIG. 2, the flow analyzer is an oil in water analyzer 20 and the sampler is a wafer sampler 22 having a metal ring 23 and a sampling line 27 penetrating the metal ring such that an opening of the sampling line is inside the metal ring. In operation, the wafer sampler 22 is mounted between two flanges of the process flow line so that the process fluid flows through the metal ring 23. The oil in water analyzer 20 may be used in the environment in which the flow analyzer 10 is used in FIG. 1. The wafer sampler 22 is configured to deliver process fluid from the process flow line to the oil in water analyzer 20. In particular, the sample line 27, which claims an inflow passage 28 and an outflow passage 29 of the wafer sampler is configured to draw fluid off from the process flow line through the inflow passage 28 and send it to the oil in water analyzer 20 and then return it to the process flow line through the outflow passage 29 at the same location in the process flow line. Preferably the passages are arranged coaxially so that the inflow passage 28 is in the form of an annulus around the outflow passage 29 or vice versa.

The shut off valve 24 connects the inflow passage 28 of the sample line 27 to inflow pipe 21 and valve 26 connects the outflow passage 29 of the sample line 27 to outflow pipe 25. Valves 24 and 26 are configured to open when the oil in water analyzer 20 is present and operating to complete an analysis flow loop line that extends from the inflow passage 28 of sample line 27 of the wafer sampler 22, to inflow pipe 21, through the oil in water analyzer 20, and back through output pipe 25 to outflow passage 29 of sample line 27. The shut off valves 24 and 26 are also be configured to be closed when the oil in water analyzer is disengaged from the flow analysis system.

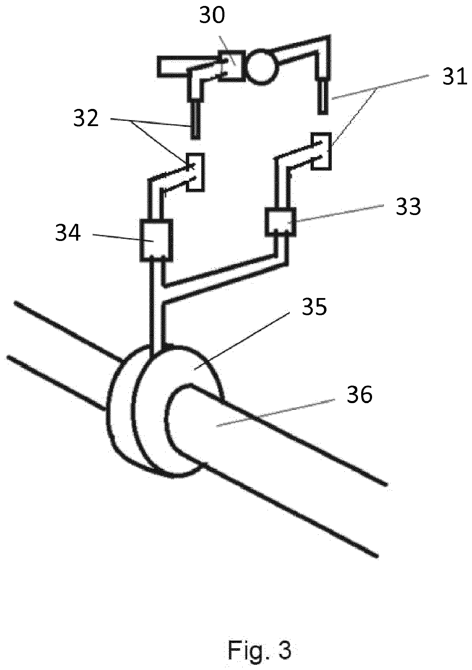

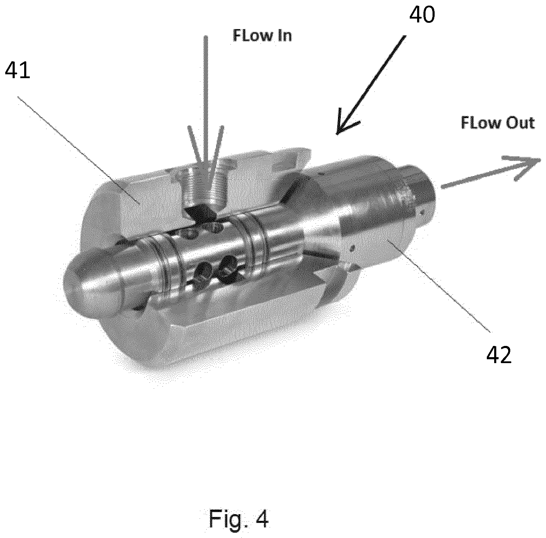

Fig. 3 shows a flow analyzer connected to a main flow line via hot stab connectors, shut off valves, and a sampler. The flow analyzer 30 depicted in Fig 3 can be, e.g., an oil in water analyzer 20 such as that depicted in FIG. 2 and can be used in the environment in which the flow analyzer 10 is used in FIG. 1. The flow analyzer 30 is connected to the main flow line 36 via sampler 35, shut off valves 33 and 34, and hot stab connectors 31 and 32. Hot stab connectors 31 and 32 can be, e.g., the type of hot stab connectors depicted in greater detail in FIG. 4. An analysis flow loop extends from the main flow line 36 through the sampler 35, through the shut off valve 33 and the hot stab connector 31 to the flow analyzer 30 and back to the main flow line 36 through the hot stab connector 32, through the shut off valve 34, and through the sampler 35. The analysis flow loop also includes all lines attached to the flow analyzer 30, the hot stab connectors 31 and 32, the shut off valves 33 and 34, and the sampler 35.

In Fig. 3, the sampler 35 is a single point sampler and can be, e.g., a single point sampling loop, an inline wafer sampler, a metallic wafer style sampling valve, and an annular probe. The sampler 35 is configured to deliver process fluid from the main flow line 36 to the flow analyzer 30. The flow analyzer 30 can be constructed with a reinforced structure sufficient to withstand the conditions of a subsea environment at depths of up to 15,000 feet. The flow analyzer 30 may include one or more spray rings for cleaning internal lenses. The hot stab connectors 31 and 32 are male and female two-piece type pipe connectors. When the male and female pieces are connected, the hot stab connectors 31 and 32 provide a flow path, e.g. for process fluid, that is sealed off from the outside environment. The shut off valves 33 and 34 can be configured to open when the flow analyzer 30 has been engaged via the hot stab connectors 31 and 32 to complete the analysis flow loop line. The shut off valves 33 and 34 can also be configured to be closed when the flow analyzer 30 is disengaged from the hot stab connectors 31 and 32.

The flow analyzers described herein, such as the oil-in-water analyzer 20 depicted in FIG. 2 and the flow analyzer 30 depicted in FIG. 3 can be constructed so as to have, in combination with pipes and connects connected thereto, a balanced weighting in order to facilitate installation via an ROV. More specifically, the flow analyzers can have a balanced weighting about a point on the flow analyzer at which the ROV is configured to connect with, or grasp, the flow analyzer. For example, the torque caused by the weight of the flow analyzer on either side of the point on the flow analyzer at which the ROV is configured to connect with the flow analyzer can be equalized through balanced weighting of the flow analyzer and the pipes and connectors connected thereto.

Fig. 4 shows a hot-stab connector. The hot stab connector 40 includes a female fitting 41 and a male fitting 42. The male fitting 42 is configured to be inserted into the female fitting 41. The flow through the hot stab connector 40 travels from the female fitting into the male fitting 40 at a ninety degree angle with respect to the direction in which the male fitting 42 is inserted into the female fitting 41. The flow through the hot stab connector 40 exits from the base of the male fitting. The flow can also be reversed in relation to that in which it is shown in Fig. 4.

While the invention has been illustrated and described in detail in the drawings and foregoing description, such illustration and description are to be considered illustrative or exemplary and not restrictive. It will be understood that changes and modifications may be made by those of ordinary skill within the scope of the following claims. In particular, the present invention covers further embodiments with any combination of features from different embodiments described above and below.

The terms used in the claims should be construed to have the broadest reasonable interpretation consistent with the foregoing description. For example, the use of the article "a" or "the" in introducing an element should not be interpreted as being exclusive of a plurality of elements. Likewise, the recitation of "or" should be interpreted as being inclusive, such that the recitation of "A or B" is not exclusive of "A and B," unless it is clear from the context or the foregoing description that only one of A and B is intended. Further, the recitation of "at least one of A, B and C" should be interpreted as one or more of a group of elements consisting of A, B and C, and should not be interpreted as requiring at least one of each of the listed elements A, B and C, regardless of whether A, B and C are related as categories or otherwise. Moreover, the recitation of "A, B and/or C" or "at least one of A, B or C" should be interpreted as including any singular entity from the listed elements, e.g., A, any subset from the listed elements, e.g., A and B, or the entire list of elements A, B and C.

## Claims

1. A flow analysis system for analyzing fluid from a process flow line (122), the system comprising:
a flow analyzer (10, 20, 30);
a single point sampler (22, 35) configured to connect to the process flow line (122), the single point sampler (22, 35) including a sample line (27) having an inflow passage (28) and an outflow passage (29);
an inflow pipe (21) connecting the inflow passage (28) of the sample line (27) to the flow analyzer (10, 20, 30); and
an outflow pipe (25) connecting the flow analyzer (10, 20, 30) to the outflow passage (29) of the sample line (27).

2. The flow analysis system of claim 1, further comprising an inflow shutoff valve (24, 33) disposed between the inflow passage (28) of the sample line (27) and the inflow pipe (21) and an outflow shutoff valve (26, 34) disposed between the outflow pipe (25) and the outflow passage (29) of the sample line (27).

3. The flow analysis system of claim 1 or 2, wherein the inflow pipe (21) includes first inflow pipe proximate the inflow shutoff valve (24, 33) and a second inflow pipe downstream of the first inflow pipe, and a first hot stab connector (31) disposed between the first and second inflow pipes.

4. The flow analysis system of one of the preceding claims, wherein the outflow pipe (25) includes first outflow pipe proximate the outflow shutoff valve (26, 34) and a second outflow pipe downstream of the first outflow pipe, and a second hot stab connector (32) disposed between the first and second outflow pipes.

5. The flow analysis system of one of the preceding claims, wherein the flow analyzer (10, 20, 30) includes one or more spray rings configured for cleaning internal lenses.

6. The flow analysis system of one of the preceding claims, wherein the flow analyzer (10, 20, 30) is constructed with a reinforced structure capable of withstanding a subsea environment of a depth of up to 15,000 feet.

7. The flow analysis system of claim 2 or 4, wherein the inflow shut off valve (24, 33) and the outflow shut off valve (26, 34) are configured to prevent flow line leakage from the process flow line (122) when the flow analyzer (10, 20, 30) is not connected to the single point sampler (22, 35).

8. The flow analysis system of one of the preceding claims, wherein the flow analyzer (10, 20, 30) has a balanced weighting.
